# EUROPÄISCHE PATENTSCHRIFT

(11) **EP 0 279 028 B1**
(45) Veröffentlichungstag und Bekanntmachung des Hinweises auf die Patenterteilung: **28.07.1993**
(21) Anmeldenummer: 87115896.0
(22) Anmeldetag: 29.10.1987
(51) Int. Cl.: A61M 5/14, A61M 25/00, F16K 15/14

(54) **Vorrichtung zur Verabreichung von Flüssigkeit**
Liquid delivery device
Dispositif pour l'administration de liquides

(30) Priorität: 19.02.1987 DE 3705357
(43) Veröffentlichungstag der Anmeldung: 24.08.1988
(73) Patentinhaber: Kabi Pharmacia GmbH, D-91052 Erlangen (DE)
(72) Erfinder: Iwatschenko, Peter, D-8524 Neunkirchen (DE)
(74) Vertreter: Behrens, Dieter, Dr.-Ing.

(56) Entgegenhaltungen:
- FR-A- 2 119 367
- US-A- 4 210 173
- US-A- 4 354 492
- US-A- 4 535 820

## Beschreibung

Die Erfindung betrifft eine Vorrichtung zur parenteralen Verabreichung von Flüssigkeit.

Bei der parenteralen Ernährung eines Patienten werden die Nährstoffe unter Umgehung des Magen/Darm-Traktes direkt in eine Vene gegeben. Neben Nährstoffen können auf diese Weise auch kontinuierlich Arzneimittel in den Blutkreislauf gebracht werden.

Vorrichtungen für die parenterale Verabreichung von Flüssigkeiten weisen im allgemeinen eine Vorratsflasche für die Flüssigkeit auf, aus welcher die Flüssigkeit dosiert über einen elastischen Schlauch zu einer Injektionsnadel geführt wird, die direkt in eine Vene mündet. Die Förderung der Flüssigkeit durch den Schlauch erfolgt über eine Pumpe und/oder die Schwerkraft der Flüssigkeit.

Vorrichtungen zur parenteralen Verabreichung von Flüssigkeiten müssen zum Schutz des Patienten hohen Sicherheitsanforderungen genügen. Insbesondere muß unter allen Umständen gewährleistet sein, daß die Förderrate, also die dem Patienten pro Zeiteinheit verabreichte Flüssigkeitsmenge, welche üblicherweise in Volumen pro Zeiteinheit oder Tropfen pro Zeiteinheit angegeben wird, einen vorgegebenen Grenzwert nicht überschreitet. Eine den Grenzwert überschreitende Mehrförderung könnte tödlich sein. Daneben darf auf keinen Fall Luft in die Vene des Patienten gelangen.

Herkömmliche Vorrichtungen zur parenteralen Verabreichung von Flüssigkeiten weisen deshalb einen hohen Aufwand an Sicherheitseinrichtungen auf. Die Sicherheitseinrichtungen betreffen in erster Linie die sogenannten "ersten Fehler". Unter einem "ersten Fehler" wird das Versagen eines Schutz- oder Überwachungssystems verstanden, das zu einer unmittelbaren Gefahr für die Sicherheit des Patienten werden kann.

Dringt Luft in den Zuführschlauch, so muß die Vorrichtung innerhalb einer Zeitspanne die Förderung von Flüssigkeit in die Vene einstellen, die kürzer ist als die Zeitspanne, in der die Luft zur Injektionsnadel gelangt. Die Zeitspanne vom Zeitpunkt des Auftretens eines Fehlers, wie z.B. das Eindringen von Luft in den Schlauch, bis zum Eintreten des sicheren Zustandes, also z.B. der Beendigung der Förderung, wird "Fehlerreaktionszeit" genannt. Die Fehlerreaktionszeit muß in jedem Fall so kurz sein, daß eine Gefährdung des Patienten bei Auftreten des Fehlers ausgeschlossen ist.

Zur Vermeidung von Luft-Förderung sind im Stand der Technik Luftsensoren bekannt, die Alarm geben, sobald Luft im zum Patienten führenden Schlauch auftritt. Ultraschalleinrichtungen und Lichtschranken sind als Luftsensoren bekannt. Diese Vorrichtungen sind relativ aufwendig und haben überdies den Nachteil, daß sie nur "diagnostizieren", also nicht positiv das Eintreten von Luft verhindern, und somit die Verfügbarkeit der Vorrichtung nicht verbessern.

Besonders beim Einsatz einer Förder-Pumpe im Zuführschlauch tritt das Problem des Lufteintritts in den Schlauch auf. Solche Pumpen sind z.B. aus der DE-PS 31 38 267 bekannt. In ihnen werden Silikon-Schläuche eingesetzt, wobei allerdings das Silikon für Luft permeabel ist, so daß dann, wenn der Druck im Inneren des Schlauches kleiner ist als der Druck in der umgebenden Luft (Atmosphäre), Gase in das Schlauchinnere eindringen können, was die bereits geschilderten fatalen Folgen hätte.

Bei bekannten Vorrichtungen zur parenteralen Verabreichung von Flüssigkeiten kann ein Unterdruck im von der Förder-Pumpe beaufschlagten Silikonschlauch durch zwei Ursachen bedingt sein: Zum einen kann bei einer Okklusion des Schlauches im Bereich der Pumpe die abwärts in Richtung auf den Patienten "hängende" Flüssigkeitssäule einen Unterdruck, insbesondere im oberen Teil des Schlauches unmittelbar unterhalb der Okklusionsstelle erzeugen und zum anderen kann es in bestimmten Arbeitsphasen der Pumpe, auf die hier nicht näher eingegangen zu werden braucht, jeweils kurzzeitig periodisch zu einem Unterdruck im Silikonschlauch stromab der Pumpe kommen.

Aus der US-A 4 210 173 ist eine Injektionsspritze für medizinische Flüssigkeiten bekannt, die ein Einlaßventil und ein Auslaßventil aufweist. Jedes Ventil hat eine flexible scheibenförmige Membran, die auf einer Seite von einem mittig angreifenden Dorn gegen einen auf der gegenüberliegenden Seite angeordneten, ringförmigen Ventilsitz gedrückt wird, so daß die Membran gegen den Ventilsitz vorgespannt ist. Vorzugsweise ist die Membran des Auslaßventils stärker vorgespannt als die Membran des Einlaßventils, so daß die beim Ansaugen von Flüssigkeit zu überwindende Kraft reduziert ist.

Der Erfindung liegt die Aufgabe zugrunde, eine Vorrichtung zur parenteralen Verabreichung von Flüssigkeit, bei der die Flüssigkeit mittels einer Pumpe durch einen elastischen Schlauch und ein stromab der Pumpe im Schlauch angeordnetes Ventil zum Patienten gefördert wird, derart weiterzubilden, daß das Ventil auch ohne Pumpe in einer lediglich schwerkraftgespeisten Vorrichtung zur parenteralen Verabreichung von Flüssigkeit einsetzbar ist. Bei einem Einsatz des Ventils mit der Pumpe soll gewährleistet sein, daß keine Luft in den Schlauch eintritt.

Erfindungsgemäß ist diese Aufgabe dadurch gelöst, daß an dem Ventil eine Einrichtung vorgesehen ist, mit der zwei unterschiedliche Öffnungsdrücke des Ventils einstellbar sind.

Bei der erfindungsgemäßen Vorrichtung ist gewährleistet, daß der Druck im Inneren des Schlauches weder durch den Sog einer "hängenden Flüssigkeitssäule" noch durch bestimmte Arbeitsphasen der Pumpe, in denen sie kurzzeitig gegen die Förderrichtung ansaugt, kleiner ist als der Stickstoff-Partialdruck der den Schlauch umgebenden Atmosphäre. Ein Eindringen von Gasen in den Schlauch ist somit positiv ausgeschlossen, da im Silikon-Schlauchsegment bis zum Ventil immer ein hinreichend großer Druck herrscht.

Auch wird durch diese Ausbildung der sogenannte "freeflow" (das freie Durchlaufen der Flüssigkeit durch den Schlauch in die Vene) verhindert.

Durch die mittels der Einrichtung einstellbaren unterschiedlichen Öffnungsdrücke kann das Ventil ersatzweise, etwa bei einem Versagen der Pumpe, auch in einer nur schwerkraftgespeisten Vorrichtung zur parenteralen Verabreichung von Flüssigkeit eingesetzt werden.

In einer bevorzugten Ausgestaltung der Erfindung ist vorgesehen, daß die Einrichtung bei Einsatz des Ventils mit einer Förderpumpe mit dieser mechanisch so gekoppelt ist, daß der höhere der beiden Öffnungsdrücke eingestellt ist.

Das durch die Erfindung bereitgestellte Ventil ist auch unabhängig vom vorstehend beschriebenen Einsatz bei der parenteralen Verabreichung von Flüssigkeiten für andere medizinische Zwecke einsetzbar und zeichnet sich durch einen einfachen Aufbau und damit eine kostengünstige Herstellung sowie eine hohe Funktionssicherheit aus. Das Ventil ermöglicht die Einstellung von extrem kleinen Öffnungsdrucken.

Aus der EP 0182045 ist zwar ein Rückschlag-Ventil bekannt, mit dem sehr kleine Öffnungsdrucke möglich sind, doch wird dort die elastische Membran auf ihrer vom Vertilsitz abgekehrten Seite von zwei Dornen beaufschlagt. Die Herstellung eines mit zwei Dornen versehenen Ventilteiles ist aufwendig und überdies erfordert der Ventil-Aufbau gemäß der EP 182045 eine zweilagige Membran.

Das erfindungsgemäße Ventil, bei dem die Membran auf ihrer vom Ventilsitz abgekehrten Seite zumindest annähernd zentrisch von einem einzigen Dorn beaufschlagt ist, zeichnet sich dadurch aus, daß in jedem Betriebszustand (also bei offenem oder geschlossenem Ventil) immer eine Zwei-Punkt- Lagerung gewährleistet ist, so daß keine radiale Verschiebung der Membran möglich ist. Die Membran bleibt also zentriert und kann nicht mit den sie radial umgebenden Wandungen in Eingriff kommen, was zu Reibung und Funktionsbeeinträchtigungen führen könnte.

Das erfindungsgemäße Ventil gewährleistet auch bei geringsten Druckdifferenzen im Bereich von wenigen Zentimetern Wassersäule ein einwandfreies Funktionieren sowohl bezüglich der Öffnungsfunktion als auch bezüglich der Schließfunktion.

In einer bevorzugten Ausgestaltung des Rückschlag-Ventils ist vorgesehen, daß der zentrale Dorn axial verschiebbar ist, so daß die Vorspannung der Membran und damit der Öffnungsdruck des Ventils veränderbar ist.

Es ist auch möglich, zwischen dem Dorn und der Membran eine Führung vorzusehen, beispielsweise in Form eines axialen Stiftes auf der Membran, der axial in eine Ausnehmung des Dornes eindringt, so daß ein Verrutschen der Membran in radialer Richtung positiv ausgeschlossen ist.

Nachfolgend werden Ausführungsbeispiele der Erfindung anhand der Zeichnung näher erläutert. Es zeigt:
- Fig. 1: schematisch eine Vorrichtung zur parenteralen Verabreichung von Flüssigkeit;
- Fig. 2: ein bei einer Vorrichtung gemäß Fig. 1 eingesetztes Ventil, das mit einer Pumpe gekoppelt ist;
- Fig. 3: ein Ventil gemäß Fig. 2, welches ohne Pumpe eingesetzt wird; und
- Fig. 4: sowie Fig. 5 abgewandelte Ausführungsformen des Ventils.

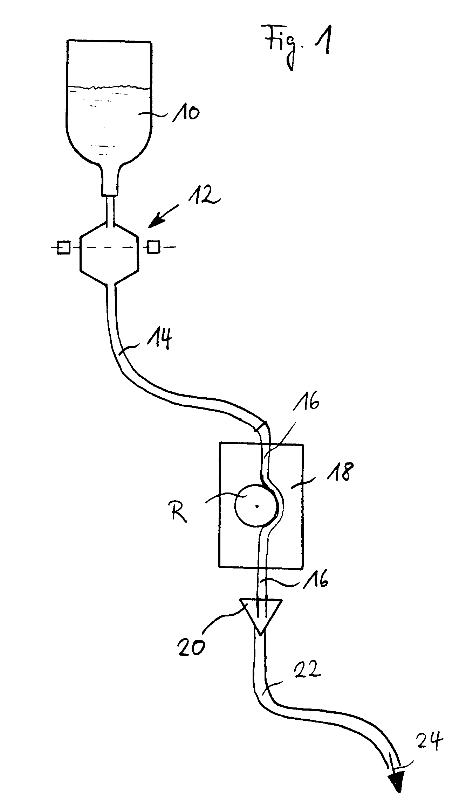

Fig. 1 zeigt eine Flasche 10, in der eine zu verabreichende Flüssigkeit, wie ein Nährmittel oder eine Arznei, enthalten ist. Mit einem Tropfensensor 12 herkömmlicher Bauart wird die Förderrate überwacht. Mittels eines elastischen Schlauches 14 aus PVC wird die Flüssigkeit im wesentlichen vertikal abwärts, also in Richtung der Schwerkraft, zur Pumpe 18 geführt. Das durch die Pumpe 18 führende Schlauchsegment 16 ist im Unterschied zum Schlauch 14 aus Silikon. Stromab der Pumpe 18 ist ein Ventil 20 angeordnet, welches weiter unten näher beschrieben werden wird. An das Ventil 20 schließt ein weiterer Schlauch 22 an, der wie der Schlauch 14 aus PVC besteht und in Richtung des Pfeiles 24 zum Patienten (nicht gezeigt) führt. Bis auf das Ventil 20 ist die in Fig. 1 gezeigte Vorrichtung von herkömmlicher Art.

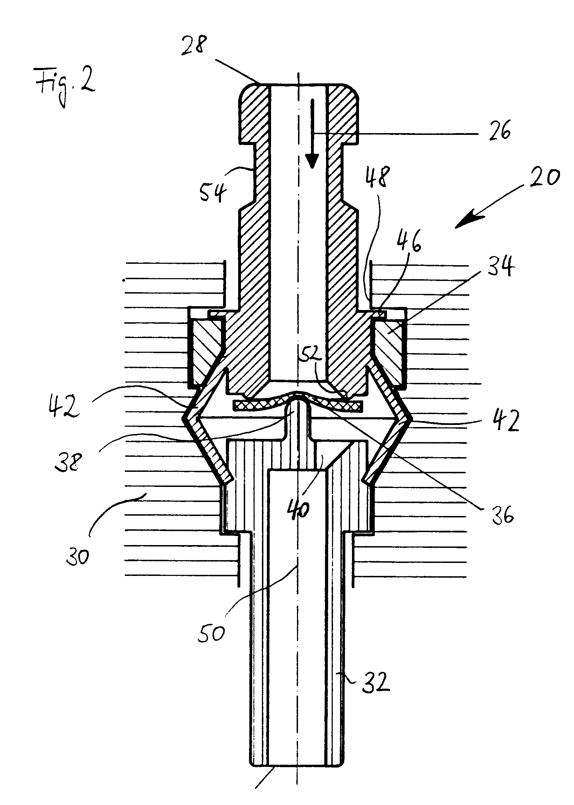

Fig. 2 zeigt das in einer Vorrichtung gemäß Fig. 1 verwendete Ventil 20 im Detail. Die Strömungsrichtung der Flüssigkeit durch das Ventil 20 ist durch der Pfeil 26 angedeutet.

Am Schlauchsegmentanschluß 28 ist stromauf das Schlauchsegment 16 aus Silikon angeschlossen (in Fig. 2 nicht gezeigt, siehe Fig. 1).

In Fig. 2 ist mit dem Bezugszeichen 30 und durch horizontale Schraffur ein Teil 30 der Pumpe 18 angedeutet. In dieses Teil 30 der Pumpe 18 wird das Ventil 20 gemäß Fig. 2 eingeschoben. Mittels eines Schlauchanschlusses 32 wird der in Fig. 1 mit dem Bezugszeichen 22 versehene PVC-Schlauch an das Ventil 20 angeschlossen.

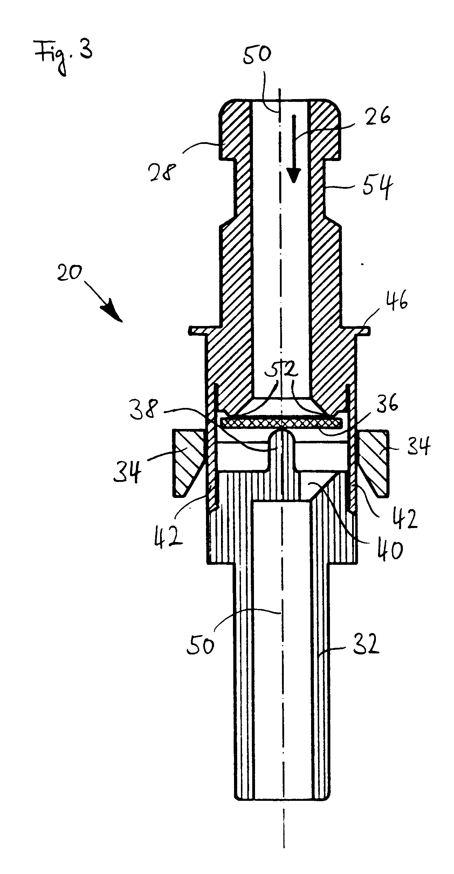

Einzelheiten des Ventils 20 sind den Fig. 2 und 3 zu entnehmen, wobei Fig. 2 das Ventil in mit dem Pumpenteil 30 mechanisch gekoppeltem Zustand zeigt, während Fig. 3 das Ventil in einem Zustand zeigt, in dem es ohne Pumpe einsetzbar ist.

Eine Einrichtung in Form eines Schieberings 34 ist axial verschiebbar und befindet sich bei dem in Fig. 2 gezeigten Zustand in einer oberen Stellung. Eine das Öffnungs- und Schließorgan des Ventils bildende Membran 36 aus Silikon wird zentrisch von einem Dorn 38 beaufschlagt. Der Öffnungsdruck des Ventils 20 wird durch die Elastizität der Membran 36 bestimmt. Übersteigt die Druckdifferenz in Richtung des Pfeiles 26 an der Membran 36 deren Andrückkraft gegen den Ventilsitz 52, so verbiegt sich die Membran 36 aus der in Fig. 2 gezeigten Schließstellung heraus nach unten und öffnet einen Durchlaß über den Kanal 40 in das Schlauchanschlußstück 32. Beim in Fig. 2 gezeigten Einbauzustand in das Pumpenteil 30 ist ein Balg 42 des Ventils 20 notwendig aufgrund einer formschlüssigen Anpassung an das Pumpenteil 30 in der gespreizten Stellung, in welcher der Dorn 38 im Vergleich mit dem in Fig. 3 gezeigten Zustand axial in Richtung auf die Membran 36 verschoben ist und die Membran mit einer größeren Kraft gegen den Ventilsitz 52 drückt, so daß der Öffnungsdruck des Ventils beim in Fig. 2 gezeigten Zustand größer ist als beim Zustand gemäß Fig. 3.

Neben dem Balg 42 ist auch der Schiehering 34 derart formschlüssig in das Pumpenteil 30 eingepaßt, daß er den Balg 42 in die Spreizlage freigibt. Der Schiebering 34 stößt dabei oben gegen einen Flansch 46 des Schlauchsegmentanschlusses 28, welcher seinerseits zwischen dem Schiebering 34 und einem Anschlag 48 des Pumpenteiles 30 eingeklemmt ist.

Am oberen Teil des Schlauchsegmentanschlusses 28 ist eine Ausnehmung 54 vorgesehen, in welcher das aufgeschobene Silikon-Schlauchsegment 16 mittels eines Sicherungsringes befestigbar ist.

Beim in Fig. 3 gezeigten Zustand des Ventils 20 sichert der Schiebering 34 die Streckstellung des Balges 42, wodurch der Dorn 38 in Richtung der Ventilachse 50 abwärts geschoben ist und die Andrückkraft der Membran 36 gegen den Ventilsitz 52 im Vergleich mit der in Fig. 2 gezeigten Stellung verringert ist.

Im in Fig. 3 gezeigten Zustand kann das Ventil 20 bei der Parenteralen Verabreichung von Flüssigkeit ohne Verwendung einer Pumpe 18 verwendet werden, wobei die Flüssigkeit nur durch ihre eigene Schwerkraft gefördert wird.

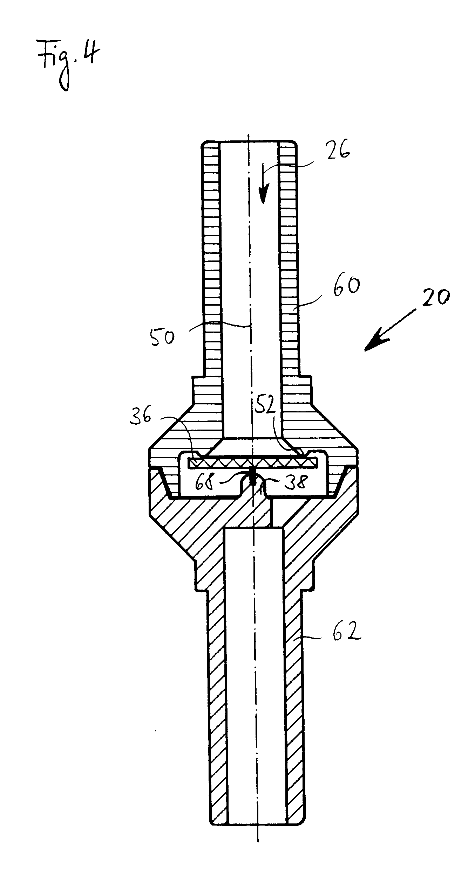

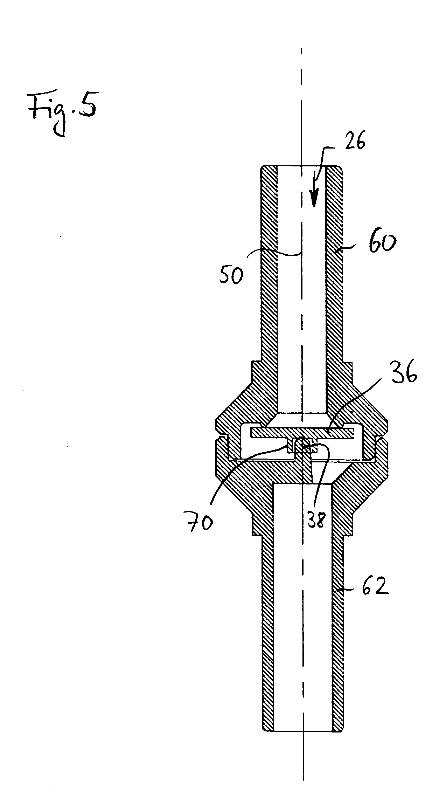

In den Fig. 4 und 5 sind weitere Ausführungsformen der Membran 36 und des Dorns 38 geseigt, wobei der Einfachheit halber auf eine Darstellung der Einrichtung 34, mittels derer die beiden unterschiedlichen Öffnungsdrücke einstellbar sind, versichtet wurde. Dem Ausführungsbeispiel gemäß den Fig. 2 und 3 entsprechende Bauteile sind mit gleichen Bezugszeichen versehen. Das in Fig. 4 gezeigte Ventil weist ein Oberteil 60 und ein Unterteil 62 auf. Am Oberteil 60 ist der ringförmige Ventilsitz 52 ausgeformt, während am Unterteil 62 der Dorn 38 ausgebildet ist. Sowohl im Schließzustand als auch im Öffnungszustand ist gewährleistet, daß die Membran 36 auf zwei radial beabstandeten Punkten gelagert ist, so daß eine radiale Verschiebung der Membran in Richtung auf die benachbarten Wände vermieden ist. Beim in Fig. 4 gezeigten Ausführungsbeispiel ist zusätzlich zu dieser Sicherung der Membran noch ein Stift 68 auf der Membran 36 vorgesehen, welcher in eine komplementäre Ausnehmung im Dorn 38 eingreift und so die Membran 36 zuverlässig zentriert.

Das in Fig. 4 gezeigte Ventil ist allgemein für medizinische Zwecke verwendbar, insbesondere für die Verabreichung von Flüssigkeiten.

In Fig. 5 ist, die Membran 36 mit einem ringförmigen Vorsprung 70 versehen, der eine Ausnehmung bildet, in welche der Dorn 38 eingreift, um die Membran in zentrischer Stellung zu sichern.

## Patentansprüche

1. Vorrichtung zur parenteralen Verabreichung von Flüssigkeit mit einer die Flüssigkeit durch einen elastischen Schlauch (14, 16, 22) zum Patienten fördernden Pumpe (18), wobei stromab der Pumpe (18) ein Ventil (20) im Schlauch (16, 22) angeordnet ist, das in Patientenrichtung (24) unter einem Druck öffnet, der so groß ist, daß der Druck im Schlauch vor dem Ventil mindestens so groß ist wie der äußere Luftdruck,
dadurch **gekennzeichnet,** daß an dem Ventil (20) eine Einrichtung (34) vorgesehen ist, mit der zwei unterschiedliche Öffnungsdrucke des Ventils (20) einstellbar sind.

2. Vorrichtung nach Anspruch 1,
dadurch **gekennzeichnet,** daß die Einrichtung (34) bei Einsatz des Ventils (20) mit der Pumpe (18) mit dieser mechanisch so gekoppelt ist, daß der höhere der beiden einstellbaren Öffnungsdrücke wirksam ist.

3. Vorrichtung nach einem der Ansprüche 1 oder 2,
dadurch **gekennzeichnet,** daß das Ventil (20) eine gegen seine Öffnungsrichtung vorgespannte, elastische Membran (36) aufweist, wobei der Öffnungsdruck in Abhängigkeit von der Vorspannung variierbar ist.

4. Vorrichtung nach Anspruch 3,
dadurch **gekennzeichnet,** daß die Membran (36) plättchenförmig ist und im Schließzustand des Ventils (20) auf einem kreisförmigen Ventilsitz (52) aufliegt.

5. Vorrichtung nach einem der Ansprüche 3 oder 4,
dadurch **gekennzeichnet,** daß die Membran (36) auf ihrer vom Ventilsitz (52) abgekehrten Seite zumindest annähernd zentrisch von einem Dorn (38) beaufschlagt ist, der längs der Ventilachse (50) verschiebbar und in verschiedenen Stellungen arretierbar ist.

6. Vorrichtung nach Anspruch 5,
dadurch **gekennzeichnet,** daß die Membran (36) von dem Dorn (38) mittels einer Führung in zentrischer Lage gehalten wird.

7. Vorrichtung nach einem der Ansprüche 3 bis 6,
dadurch **gekennzeichnet,** daß die Membran (36) einstückig aus Silikon besteht.

8. Vorrichtung nach einem der Ansprüche 3 bis 7,
dadurch **gekennzeichnet,** daß die Membran (36) mit einer Ausnehmung oder einem ringförmigen Vorsprung (70) versehen ist, in welche bzw. welchen ein Dorn (38) eingreift.

## Claims

1. Apparatus for parenteral administration of liquid comprising a pump (18) conveying the liquid through an elastic tube (14, 16, 22) to the patient, a valve (20) being arranged downstream of the pump (18) in the tube (16, 22) and opening in the direction (24) of the patient under a pressure which is so large that the pressure in the tube in front of the valve is at least as great as the external air pressure,
characterized in that on the valve (20) a means (34) is provided with which two different opening pressures of the valve (20) can be set.

2. Apparatus according to claim 1,
characterized in that when using the valve (20) with the pump (18) the means (34) is coupled to said pump mechanically in such a manner that the higher of the two settable opening pressures is effective.

3. Apparatus according to claim 1 or 2,
characterized in that the valve (20) comprises an elastic diaphragm (36) biased against its opening direction, the opening pressure being variable in dependence upon the bias.

4. Apparatus according to claim 3,
characterized in that the diaphragm (36) is plate-like and in the closure state of the valve (20) bears on a circular valve seat (52).

5. Apparatus according to claim 3 or 4,
characterized in that on its side remote from the valve seat (52) the diaphragm (36) is acted upon at least approximately centrally by a mandrel (38) which is displaceable along the valve axis (50) and lockable in various positions.

6. Apparatus according to claim 5,
characterized in that the diaphragm (36) is held by the mandrel (38) by means of a guide in a central position.

7. Apparatus according to any one of claims 3 to 6,
characterized in that the diaphragm (36) is made integrally from silicone.

8. Apparatus according to any one of claims 3 to 7,
characterized in that the diaphragm (36) is provided with a recess or an annular projection (70) into which a mandrel (38) engages.

## Revendications

1. Dispositif pour administrer un liquide par voie parentérale, comportant une pompe (18) envoyant au patient le liquide à travers un tuyau (14, 16, 22) élastique, dans lequel une soupape (20) est montée dans le tuyau (16, 22), en aval de la pompe (18), laquelle soupape s'ouvre en direction du patient (24), sous une pression qui est suffisamment grande pour que la pression dans le tuyau en amont de la soupape soit au moins égale à la pression atmosphérique extérieure, caractérisé en ce qu'il est prévu sur la soupape (20) un dispositif (34) permettant de régler deux pressions d'ouverture différentes de la soupape (20).

2. Dispositif selon la revendication 1, caractérisé en ce que le dispositif (34) est accouplé mécaniquement avec la pompe (18), dans le cas d'une utilisation de la soupape (20) avec cette pompe, de manière qu'agisse la plus grande des deux pressions d'ouverture réglables.

3. Dispositif selon l'une des revendications 1 ou 2, caractérisé en ce que la soupape (20) présente une membrane (36) élastique, précontrainte dans le sens contraire à son sens d'ouverture, la pression d'ouverture pouvant varier en fonction de la précontrainte.

4. Dispositif selon la revendication 3, caractérisé en ce que la membrane (36) est en forme de plaquette et repose, en position fermée de la soupape (20), sur un siège de soupape (52) circulaire.

5. Dispositif selon l'une des revendications 3 ou 4, caractérisé en ce que la membrane (36) est sollicitée, sur son côté opposé au siège de soupape (52), de manière au moins à peu près centrée, par un pointeau (38) qui peut coulisser le long de l'axe de la soupape (50) et qui peut être bloqué dans différentes positions.

6. Dispositif selon la revendication 5, caractérisé en ce que la membrane (36) est maintenue en position centrée, par le pointeau (38), au moyen d'un organe de guidage.

7. Dispositif selon l'une des revendications 3 à 6, caractérisé en ce que la membrane (36) est réalisée d'une seule pièce, en silicone.

8. Dispositif selon l'une des revendications 3 à 7, caractérisé en ce que la membrane (36) est pourvue d'un évidement ou d'une saillie (70) annulaire dans lequel ou dans laquelle s'engage un pointeau (38).
